(19) 

(11) **EP 4 646 921 B1**

(12) **EUROPEAN PATENT SPECIFICATION**

(45) Date of publication and mention of the grant of the patent:
**30.09.2026 Bulletin 2026/40**

(21) Application number: **25165987.6**

(22) Date of filing: **25.03.2025**

(51) International Patent Classification (IPC):
***A01G 25/16*** *(2006.01)* ***G01N 33/24*** *(2006.01)*
***G06Q 50/02*** *(2024.01)*

(52) Cooperative Patent Classification (CPC):
**G01N 33/246; A01G 25/167; G06Q 50/02;**
G01N 33/0098

(54) **SYSTEM AND METHOD FOR ESTIMATING CROP WATER REQUIREMENT USING MULTI-SENSOR DATA FUSION**

SYSTEM UND VERFAHREN ZUR SCHÄTZUNG DES ERNTEWASSERBEDARFS UNTER VERWENDUNG VON MULTISENSORDATENFUSION

SYSTÈME ET PROCÉDÉ D’ESTIMATION D’EXIGENCE D’EAU DE CULTURE À L’AIDE D’UNE FUSION DE DONNÉES MULTICAPTEURS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.05.2024 IN 202421036177**

(43) Date of publication of application:
**12.11.2025 Bulletin 2025/46**

(73) Proprietor: **Tata Consultancy Services Limited Maharashtra (IN)**

(72) Inventors:
- **SAWANT, Suryakant Ashok**
**411057 Pune, Maharashtra (IN)**
- **KUMAR, Jay Prakash**
**560066 Bangalore, Karnataka (IN)**
- **MOHITE, Jayantrao**
**400601 Thane (West), Maharashtra (IN)**
- **PANDIT, Ankur**
**453112 Indore, Madhya Pradesh (IN)**
- **PAPPULA, Srinivasu**
**500034 Hyderabad, Telangana (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**US-A1- 2017 038 749** **US-A1- 2019 230 875**
**US-A1- 2021 110 157** **US-A1- 2022 061 236**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 646 921 B1

## Description

CROSS-REFERENCE TO RELATED APPLICATIONS AND PRIORITY

The present application claims priority from Indian patent application no. 202421036177, filed on May 7, 2024.

TECHNICAL FIELD

The disclosure herein generally relates to estimation of crop water requirement, and, more particularly, to system and method for estimating crop water requirement using multi-sensor data fusion.

BACKGROUND

Agriculture, industry, and urban areas are competing for available water resources. Increasing global population is imparting pressure on both agriculture for food demand and limited freshwater resources for consumption. Crop water requirements for crops are found to estimate the total quantity of water required for the crops for their full growth throughout their growing period. The water requirement of every crop is different, and it also depends upon the weather conditions of that particular area. With the advent of accurate global weather forecast models, low orbit earth observation satellites and precision irrigation technologies have opened opportunities to understand the crop growth conditions.

Crop water requirement is a function of crop characteristics, crop management, soil, and weather which is defined as depth of water required to meet the water consumed through crop metabolic activities such as evaporation and transpiration. Better crop water management results in healthy crop, growing in fields under non-restricting soil conditions including soil water and fertility, and achieving full production potential under the given growing environment.

Variations in local weather conditions, crop management practices and variable soil conditions pose challenges to accurate estimation of crop water requirement. Accurate estimate of crop water requirement leads to reduction in water and energy use. The scalability of current crop water requirement estimation models is limited by availability of accurate weather observations, weather forecasts, information about the current crop growth phase and dynamics of soil water balance. Continued evolution in the field level agricultural cropping practices and enhancements is the desired outcome of the present challenges.

US 2022/061236 A1 discloses an integrated multi-scale modeling platform is utilized to assess agricultural productivity and sustainability. The model is used to assess the environmental impacts of agricultural management from individual fields to watershed/basin to continental scales. In addition, an integrated irrigation system is developed using data and a machine-learning model that includes weather forecast and soil moisture simulation to determine an irrigation amount for farmers. Next, crop cover classification prediction can be established for an ongoing growing system using a machine learning or statistical model to predict the planted crop type in an area. Finally, a method of predicting key phenology dates of crops for individual field parcels, farms, or parts of a field parcel, in a growing season, can be established (Abstract).

US 2021/110157 A1 discloses techniques for estimating crop maturity using remote sensing imagery are disclosed. A method may include a step of providing a remote sensing image of crops acquired at an image acquisition time, for example, a synthetic aperture radar image acquired from a satellite. The method may also include a step of predicting a maturity level of the crops at the image acquisition time based on input crop maturity data from an input time and weather-based growth indication data from between the input time and the image acquisition time. The weather-based growth indication data may include growing degree day data. The method may further include a step of updating the predicted maturity level to an updated maturity level of the crops based on the remote sensing image and a response model relating remote sensing image information to crop maturity information. The predicting and updating steps may involve performing a particle filtering operation (Abstract).

SUMMARY

Embodiments of the present disclosure present technological improvements as solutions to one or more of the above-mentioned technical problems recognized by the inventors in conventional systems. For example, in one embodiment, a system for field level crop water requirement estimation is provided. The invention is described in the appended set of claims 6-10.

In another aspect, a method for estimating crop water requirement using multi-sensor data fusion is provided. The invention is described in the appended set of claims 1-5.

In yet another aspect, there are provided one or more non-transitory machine-readable information storage mediums comprising one or more instructions, which when executed by one or more hardware processors causes a method for estimating crop water requirement using multi-sensor data fusion. The invention is described in the appended

set of claims 11-15.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG.1 is a schematic view of an environment where a system is deployed for estimation of crop water requirement using multi-sensor data fusion, in accordance with some embodiments of the present disclosure.

FIG.2 is a functional block diagram of a system for estimation of crop water requirement, in accordance with some embodiments of the present disclosure.

FIG.3 (collectively referred as FIG.3A and FIG.3B) depicts a use case example for estimating crop water requirement using the system of FIG. 2, in accordance with some embodiments of the present disclosure, in accordance with some embodiments of the present disclosure.

FIG.4 (collectively referred as FIG.4A and FIG.4B) is a flow diagram illustrating a method for FIG.2 depicts process flow of the system for estimating crop water requirement, in accordance with some embodiments of the present disclosure.

FIG.5 is a visual representation of variation of air and water in soil profile at various time steps using the system of FIG.2, in accordance with some embodiments of the present disclosure, in accordance with some embodiments of the present disclosure.

FIG.6 is a visual representation of soil water balance using the system of FIG.2, in accordance with some embodiments of the present disclosure, in accordance with some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

**GLOSSARY:**

**Rainfall:** Water received by precipitation rainfall or snow.

**Irrigation:** Water provided using irrigation system using methods like flood, surface drip, sub-surface drip, sprinkler.

**Evapotranspiration (ET):** Estimate of Evaporation and Transpiration of selected crop in the field. ET is divided into two types refence ET and crop ET.

**Runoff:** Excess water that moves out from fields as flow in drains. Runoff is the part of the water cycle that flows over the land as surface water rather than being absorbed into groundwater or evaporating. It appears in uncontrolled surface streams, rivers, drains, or sewers. Factors that affect runoff include the amount of rainfall, permeability, vegetation, and the slope of the land.

**Percolation:** Excess water moving out of soil layer and can be available as capillary rise.

**Deep percolation:** Excess water moving out of soil layer and unavailable to the crop.

**Capillary rise:** Vertical movement of the water through capillary rise.

**Field Capacity:** Field capacity is the amount of water that is retained in the soil after excess water has drained away and the rate of downward movement has decreased. This usually takes place 2-3 days after rain or irrigation in pervious soils of uniform structure and texture.

**Wilting Point:** The wilting point is the soil moisture content at which plants can no longer absorb water from the soil. This occurs when the water potential in the soil is lower than the water potential in the plant roots.

**Total Available Water (TAW):** Total water stored in soil profile at field capacity and available for crop use.

**Depletion Coefficient:** Depletion coefficient is dimensionless number used to control availability of water in soil profile.

**Readily Available Water / profile moisture:** Lower level of moisture available in soil profile for crop use.

**Available profile moisture:** moisture available in soil profile at given time.

**Irrigation Event:** Day of providing the irrigation with amount of irrigation.

**Soil Depth, Root Zone Depth:** Depth of soil layer, Effective soil depth available for crop root growth.

**Crop Coefficient:** A crop coefficient is a dimensionless number that represents the ratio of the actual crop Evapotranspiration $ET_c$ to the reference Evapotranspiration $ET_o$. $ET_c$ is the amount of water that is lost from a crop through transpiration and evaporation, while $ET_o$ is the amount of water that would be lost from a well-watered reference crop, such as grass, under the same environmental conditions. Crop coefficients vary depending on the crop type, the stage of crop growth, and the environmental conditions. For example in Tea Crop Coefficient is function of harvest, pruning and shade tree density.

Water is a crucial or important factor for crop production. More than 80% of water resources have been exploited for irrigation. Water requirement of crops refers to the amount of water that is needed by a particular crop during its growth cycle to produce an optimum yield. The water requirement varies from crop to crop and is affected by various factors such as the stage of crop growth, weather conditions, soil type, and the availability of water. Understanding the water requirement of crops is crucial for farmers and agricultural experts to implement effective water management strategies and ensure sustainable crop production.

Crop water requirements (CWR) refers to the amount of water required to compensate for evapotranspiration losses from a cropped field during a specified period. The concept of crop water requirements has become important with the development of large engineering works when it was necessary to estimate the water volumes to be supplied to newly irrigated areas. Crop water requirement is required to meet the water loss through evapotranspiration of a disease-free crop growing in fields under variable soil conditions, including soil water and fertility status, and achieving its full grain production potential under the given environmental conditions (including soil water and fertility status). Identifying water requirements and crop coefficients is critical for irrigation scheduling and agricultural water management in field management.

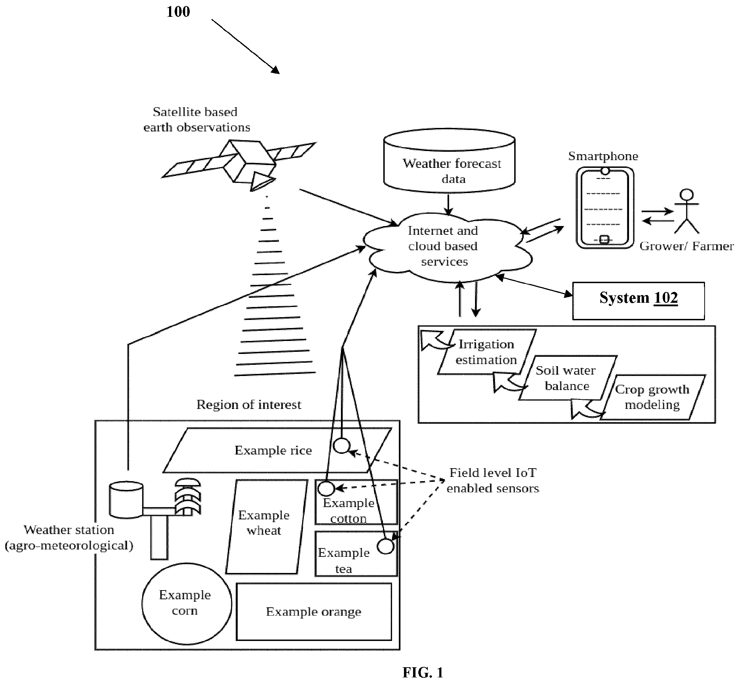

Embodiments herein provide a system and method for estimating crop water requirement using multi-sensor data fusion. The system may be alternatively referred as a water requirement estimation system. The system is a multi-layered crop and soil water balance modeling using a plurality of sensors. The method of the present disclosure divides both soil and crop into multiple vertical and horizontal profiles to estimate water balance thereby reducing the errors in estimation of crop water requirement. Observations from multiple sources such as satellite-based earth observations, regional weather observations from agro-meteorological systems, field level weather observations from IoT sensors, weather forecasts from global circulation models, and crop knowledge base is used for crop water requirement estimation. The method is based on spatio-temporal modeling for multi-layer crop and soil water balance and helps to generate the additional insights on crop water requirement like moisture at different levels in soil profile, crop canopy growth at different locations. The disclosed system is further explained with the method as described in conjunction with FIG.1 to FIG.6 below.

Referring now to the drawings, and more particularly to FIG.1 through FIG.6, where similar reference characters denote corresponding features consistently throughout the figures, there are shown preferred embodiments and these embodiments are described in the context of the following exemplary system and/or method.

FIG.1 is a schematic view of an environment where a system is deployed for estimation of crop water requirement using multi-sensor data fusion, in accordance with some embodiments of the present disclosure.

The FIG.1 depicts a plurality of fields, each field also referred to as agriculture or horticulture field of interest. Satellite based sensor captures agriculture or horticulture field data for an area of coverage. The coverage area includes the agriculture field comprising one or more growing crops, for example sugarcane, crop, wheat, rice, cotton, tea, orange, and the like for cultivating different crops. The system 102, with known in the art techniques tag the geographical location of agriculture crop fields to identify boundaries of each field of interest. Each field may have IoT enabled sensors to capture information such as soil moisture, soil temperature, air temperature, and the like. Agro-meteorological sensors capture weather data of the field under coverage transmitting the sensor information via internet to cloud based services storage. The region having agro-meteorological weather station has sensors that measure weather parameters such as temperature, relative humidity, wind speed, wind direction, rainfall, solar radiation, and the like. The weather parameters include maximum temperature and minimum temperature, maximum relative humidity, minimum relative humidity wind speed, solar radiation, and the effective rainfall. The field sensor data includes the soil moisture. The soil parameters include field capacity, wilting point, and total available water for that crop based on root zone depth. The crop parameters include the crop coefficient, crop growth stage, root zone depth, depletion coefficient and the crop area.

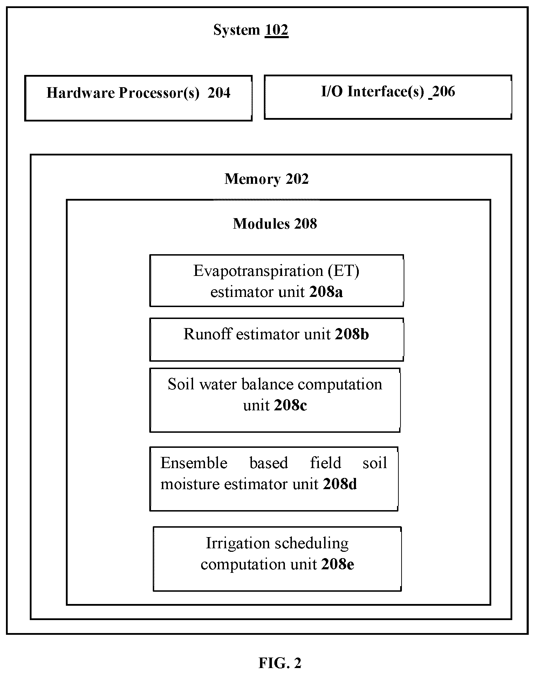

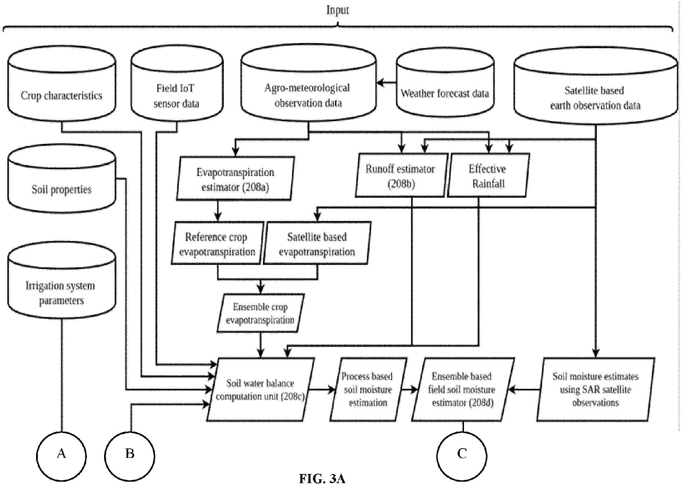

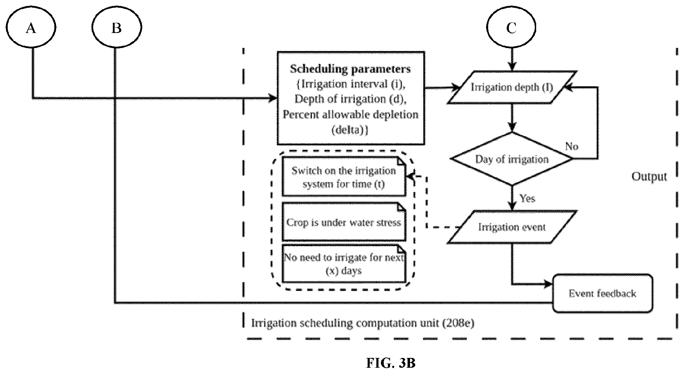

The data captured from the IoT based sensors and the agro-meteorological sensors are transmitted to cloud based data store or service which is further used by multiple stakeholders. It is noted each field may have or may not have sensor(s). Each field may not have the sensor deployed then regional agro-meteorological observations and satellite based observations utilized in the method. Additionally, each field has onsite deployed sensors that directly communicate with a cloud server and associated mobile devices through which respective farmers or landowners update information related to various events taking place on the agriculture field of interest into the cloud server. The satellite or drone based earth observation data is collected in multi-spectral, hyperspectral, thermal and Synthetic Aperture Radar (SAR) sensors. The earth observation data is available in open source and commercial modes. The earth observations data from multi-spectral or hyperspectral sensors is used for calculation of vegetation indices, whereas SAR data is used for calculation of

soil moisture (Pandit et al., 2022). Weather forecast data is obtained from process based global circulation models. The weather forecast data includes parameters such as temperature, humidity, precipitation (rainfall), solar radiation, etc. The weather forecasts of short and medium term (about seven to fifteen days) are also used for the crop water requirement analysis. The geospatial boundary of each field, crop management practices, information related to irrigation system is collected using mobile or web application installed on the Smartphone or tablet or computing device (laptop or personal computer) of the user (farmer, or stakeholder). The region specific information about soil that includes soil type, soil texture, soil depth and elevation of the field is obtained using regional soil database. The system also records the information on crops grown in the region, neighboring crop fields, weather data, and harvesting patterns and the like. The system 102 is further explained with respect to FIG.2 through FIG.6 and method depicted in flow diagram of FIG.3.

FIG.2 is a functional block diagram of a system for estimation of crop water requirement, in accordance with some embodiments of the present disclosure.

In an embodiment, the system 102 includes a processor(s) 204, communication interface device(s), alternatively referred as input/output (I/O) interface(s) 206, and one or more data storage devices or a memory 202 operatively coupled to the processor(s) 204. The system 102 with one or more hardware processors is configured to execute functions of one or more functional blocks of the system 102.

Referring to the components of the system 102, in an embodiment, the processor(s) 204, can be one or more hardware processors 204. In an embodiment, the one or more hardware processors 204 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 204 are configured to fetch and execute computer-readable instructions stored in the memory 202. In an embodiment, the system 102 can be implemented in a variety of computing systems including laptop computers, notebooks, hand-held devices such as mobile phones, workstations, mainframe computers, servers, and the like.

The I/O interface(s) 206 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, LoRA, cellular and the like. In an embodiment, the I/O interface (s) 206 can include one or more ports for connecting to a number of external devices or to another server or devices.

The memory 202 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 202 includes a plurality of modules 208 comprising a evapotranspiration estimator 208a, a runoff estimator 208b, a soil water balance computation unit 208c, an ensemble based field soil moisture estimator 208d and an irrigation scheduling computation unit 208e.

The plurality of modules 210 include programs or coded instructions that supplement applications or functions performed by the system 102 for executing different steps involved in the process of estimation of soil water balance, being performed by the system 102. The plurality of modules 210, amongst other things, can include routines, programs, objects, components, and data structures, which performs particular tasks or implement particular abstract data types. The plurality of modules 210 may also be used as, signal processor(s), node machine(s), logic circuitries, and/or any other device or component that manipulates signals based on operational instructions. Further, the plurality of modules 210 can be used by hardware, by computer-readable instructions executed by the one or more hardware processors 104, or by a combination thereof. The plurality of modules 210 can include various sub-modules (not shown).

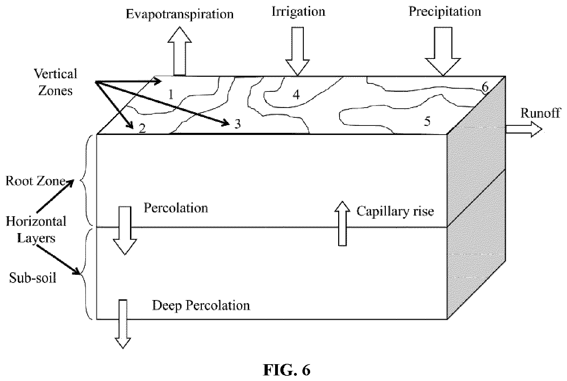

Further, the memory 102 may comprise information pertaining to input(s)/output(s) of each step performed by the processor(s) 104 of the system 102 and methods of the present disclosure. Further, the memory 202 includes a database 208. Although the database 208 is shown internal to the system 102, it will be noted that, in alternate embodiments, the database 208 can also be implemented external to the system 102, and communicatively coupled to the system 102. The data contained within such external database may be periodically updated. For example, new data may be added into the database (not shown in FIG.2) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 102 are now explained with reference to FIG.2 through steps of flow diagram in FIG.6.

FIG.3 (collectively referred as FIG.3A and FIG.3B) depicts a use case example for estimating crop water requirement using the system of FIG. 2, in accordance with some embodiments of the present disclosure, in accordance with some embodiments of the present disclosure. In an embodiment, the memory 202 includes a plurality of modules 208 comprising the evapotranspiration estimator 208a, the runoff estimator 208b, the soil water balance computation unit 208c, the ensemble based field soil moisture estimator 208d and the irrigation scheduling computation unit 208e.

The evapotranspiration estimator 208a of the system 100 estimates reference ET, crop ET and crop ET at non-standard conditions or field conditions. The ET rate from a reference surface, not short of water, is called the reference crop

ET or reference ET and is denoted as $ET_o$.

The runoff estimator 208b estimates of the system 100 land cover condition using satellite based land use land cover classification and antecedent rainfall observed by weather station.

The soil water balance computation unit 208c of the system 100 computes conceptual multi-layered (horizontal and vertical) model.

The ensemble based field soil moisture estimator 208d of the system 100 dynamically adjusts one or more parameters used for crop water requirement estimation.

The irrigation scheduling computation unit 208e of the system 100 considers entire soil profile. The soil profile comprises of solid, air and water, where the water is absorbed by roots for consumptive use and empty space is filled by air.

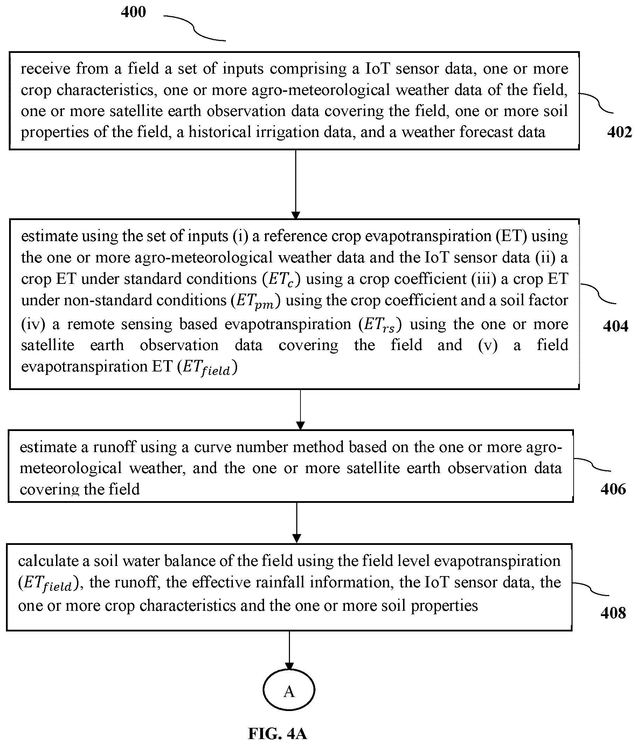

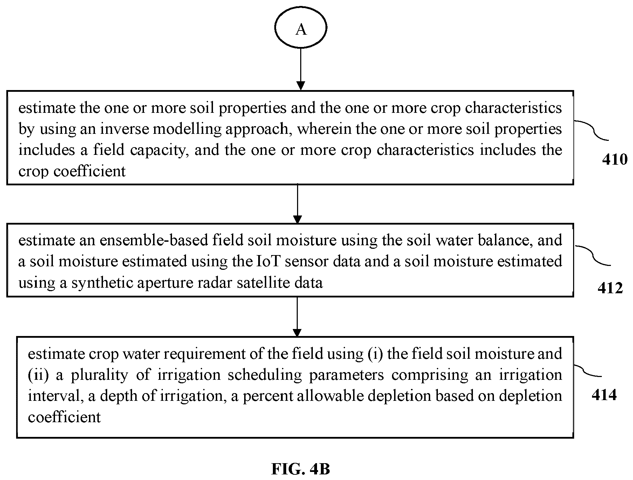

FIG.4 (collectively referred as FIG.4A and FIG.4B) is a flow diagram illustrating a method for FIG.2 depicts process flow of the system for estimating crop water requirement, in accordance with some embodiments of the present disclosure.

In an embodiment, the system 100 comprises one or more data storage devices or the memory 102 operatively coupled to the processor(s) 104 and is configured to store instructions for execution of steps of a method 400 by the processor(s) or one or more hardware processors 104. The steps of the method 400 of the present disclosure will now be explained with reference to the components or blocks of the system 100 as depicted in FIG.1 through FIG.2, and the steps of flow diagram as depicted in FIG.4. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Referring to the steps of the method 400, at step 402 a one or more hardware processor is configured to receive from a field a set of inputs comprising a IoT sensor data, one or more crop characteristics, one or more agro-meteorological weather data of the field, one or more satellite earth data covering the field, one or more soil properties of the field, a historical irrigation data, and a weather forecast data.

Referring to an example, where the system 100 receives a request from external users to estimate field level crop water requirement on daily basis. The external user may be farmer, crop grower and the like. Once the request is received, the system 100 collects the set of inputs corresponding to the field required to process the request. The field may be an agriculture or horticulture.

The IoT sensor data of the set of inputs includes a soil moisture, a soil temperature, and an air temperature.

The one or more crop characteristics of the set of inputs includes a crop root zone depth, a crop age, a crop coefficient, a percentage crop canopy cover, a crop specific wilting point moisture content, and a crop specific permanent wilting point moisture content.

The one or more agro-meteorological weather data of the field includes a temperature, a relative humidity, a wind speed, a wind direction, an rainfall information, and a solar radiation information.

The one or more satellite earth data covering the field includes multi-spectral satellite data of Sentinel 2 or Landsat 8 or Landsat 9 satellite, and synthetic aperture radar satellite data from sensors of satellite like sentinel 1.

The one or more soil properties of the field includes a soil type, a soil depth, a wilting point moisture content, a permanent wilting point moisture content, a soil porosity, and a level of water table.

The irrigation system parameters includes but not limited to type of irrigation such as surface, drip, sprinkler, micro-sprinkler, sub-surface, spacing of emitters and emitter discharge, and crop row spacing.

The historical irrigation data includes but not limited to the day of irrigation, an amount of irrigation or time of irrigation pump operation with pump capacity and irrigation details.

The weather forecast data includes but not limited to an air temperature, a relative humidity, a precipitation (rain and snow), a solar radiation and a wind speed.

At step 404 of the method 400, the one or more hardware processors is configured to estimate using the set of inputs (i) a reference crop evapotranspiration ($ET_o$) using the one or more agro-meteorological weather data and the IoT sensor data (ii) a crop ET under standard conditions ($ET_c$) using a crop coefficient (iii) a crop ET under non-standard conditions ($ET_{pm}$) using the crop coefficient and a soil factor (iv) a remote sensing based evapotranspiration ($ET_{rs}$) using the one or more satellite earth observation data covering the field and (v) a field evapotranspiration ET ($ET_{field}$). Here, the ET field is an ensemble of the remote sensing based evapotranspiration and the crop ET under non-standard conditions.

For the above example, the set of inputs are received, to estimate the reference crop evapotranspiration ($ET_o$) using the one or more agro-meteorological weather data and the IoT sensor data. The reference crop ($ET_o$) is the evapotranspiration rate from a reference surface, not short of water. The reference surface is a hypothetical grass reference crop with specific characteristics as denoted in Equation 1,

*Climate (Radiation, Temperature, Wind Speed, Humidity) + Grass Reference Crop (well watered grass) = ETo* Equation 1

The crop evapotranspiration (ET) under standard conditions ($ET_c$) is estimated using the crop coefficient. The crop ET under standard conditions disease-free, well-fertilized crops, grown in large fields, under optimum soil water conditions, achieving full production under the given climatic conditions as in Equation 2,

$$ET_c * K_c = ET_c$$

----------Equation 2,

Further, the crop ET under non-standard conditions ($ET_{pm}$) is estimated using the crop coefficient and a soil factor. The crop ET is grown under management and environmental conditions that differ from the standard conditions. When cultivating crops in fields deviate from $ET_c$ due to non-optimal conditions such as the presence of pests and diseases, soil salinity, low soil fertility, water shortage or waterlogging. This may result in scanty plant growth, low plant density and may reduce the ET rate below $ET_c$ as defined in Equation 3,

$$ET_o * (K_s * K_c) = ET_c\, adj \qquad \text{----- Equation 3}$$

Where, $K_s = f$ {biotic stress, abiotic stress}

The extent of biotic stress (due to pest/disease attack) is calculated using the field level smartphone camera images. The abiotic stress (due to temperature, moisture, etc.) is calculated using remote sensing-based vegetation indices such as Normalized Difference Vegetation Index (NDVI), Normalized Difference Water Index (NDWI) and Leaf Chlorophyll Index (LCI).

Then, the remote sensing-based ET ($ET_{rs}$) is estimated using the one or more satellite earth data covering the field. Satellite based earth observations such as multi-spectral and thermal infra-red region are used to calculate the $ET_{rs}$.

Finally, the field level ET ($ET_{field}$) is estimated using the ensemble based approach Penman-Montheith based ET ($ET_{pm}$) and the remote sensing based ET ($ET_{rs}$) are used as input and filtering techniques are used to calculate the field level ET ($ET_{field}$) as in Equation 4,

$$ET_{field} = f\{ET_{pm}, ET_{rs}\} \qquad \text{------ Equation 4}$$

At step 406 of the method 400, the one or more hardware processors is configured to estimate a runoff using a curve number method based on the one or more agro-meteorological weather, and the one or more satellite earth data covering the field.

Further for the above example, the runoff is calculated using Soil Conservation System (SCS) curve number method. Where land cover condition estimated using satellite based land use land cover classification and antecedent rainfall observed by weather station are used as input as defined in Equation 5,

$$Q = \frac{(P - I_a)^2}{(P - I_a) + S} \qquad \text{-----------------Equation 5}$$

Where, Q = Runoff (in), P = rainfall (in), S = potential maximum retention after runoff begins, $I_a$ = initial abstractions $I_a = 0.2 \times S$.

Substuting the $I_a$ value into Q to obtain the Equation 6 and 7,

$$Q = \frac{(P - 0.2\,S)^2}{(P + 0.8\,S)} \qquad \text{--------------Equation 6}$$

$$S = \frac{1000}{CN} - 10 \qquad \text{----- Equation 7}$$

(SCS Curve Number Method)

Now step 408 of the method 400, the one or more hardware processors is configured to calculate a soil water balance of the field using the field level evapotranspiration ($ET_{field}$), the runoff, the effective rainfall information, the IoT sensor data, the one or more crop characteristics and the one or more soil properties.

In one embodiment, the soil water balance approach is defined using conceptual multi-layered horizontal soil profile and a vertical soil profile. The water storage in different layers of the soil is governed by various factors such as the effective rainfall, the irrigation, the evapotranspiration, the runoff, the percolation, the deep percolation and, the capillary

rise. The storage of soil moisture ($\Delta_s$) is a function of a first set of factors and a second set of factors as in Equation 8,

$$\Delta_s = \text{the first set of factors (input)} - \text{the second set of factors (output)} \quad \text{----- Equation 8}$$

The first set of factors includes the effective rainfall, an irrigation, capillary rise, the runoff, a percolation, a deep percolation, and the evapotranspiration. The effective rainfall is obtained from the weather station observation and converted to effective rainfall (i.e., amount of water that enters the soil). Irrigation is calculated using the details of irrigation system. Capillary rise is calculated using a linear model and a soil saturation percentage. The second set of factors includes the runoff, the percolation is a component of the effective rainfall, and the deep percolation is a component of the vertical soil water movement. The runoff is calculated using the SCS curve number method as in Equation 5. The percolation is a component of the effective rainfall that defines a vertical movement of water into the soil profile. Deep percolation is a component of the vertical soil water movement where the water is lost to the subsurface and never recovered by natural process such as the capillary rise. Evapotranspiration is calculated using multiple methods such as Penman-Monteith Equations 1-3 and a METRIC model.

In one embodiment, the water balance equations are described below,

$$\begin{aligned} &Total\ Available\ Water\ (TAW) \\ &\quad = (FC - PWP) \\ &\quad * Root\ zone\ depth\ for\ that\ crop\ growth\ stage \end{aligned} \quad \text{---------------Equation 9}$$

Where, *Fc* = field capacity and *PWP* = permanent wilting point

Further, the readily available water is a sum of the total available water and depletion coefficient as described in Equation 10,

$$Readily\ Available\ Water\ (RAW) = TAW * Depletion\ coefficient \quad ---------- Equation\ 10$$

The change in storage is calculated in Equation 11,

$$DS = P + I - E_{TC} - R \quad \text{------------- Equation 11}$$

Where, *DS = Storage in soil profile also refereed as RAW*,

*P = Cumulative rainfall (mm), I = Irrigation applied (mm),* $E_{Tc}$ *= Crop Evapotranspiration, R = Runoff.*

The available soil profile moisture (APM) in Equation 12,

$$APM_{i-1} + DS_i\ (For\ 1st\ day\ to\ start\ the\ balance\ APM_{i-1} = TAW) \quad \text{------------- Equation 12}$$

Decide if $i^{th}$ day is the irrigation day in Equation 13,

$$\text{Check, } APM_i = (TAW - (RAW - 2)) \quad \text{------------- Equation 13}$$

The APM is calculated for each day till $APM_{(i)}$ reaches (TAW - (RAW-2)) and irrigation irrigation amount is calculated in mm as in Equation 14,

$$IR = \frac{TAW - APM(i)}{Irrigation\,System\,Efficiency}$$

------------- Equation 14

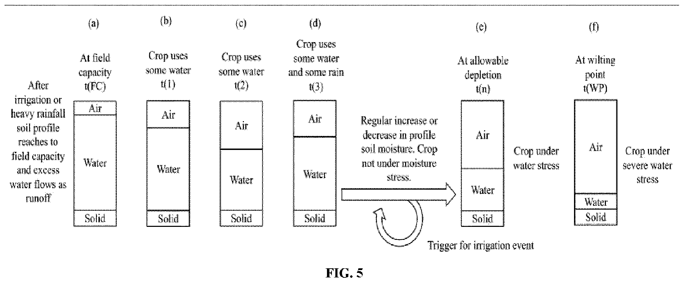

In one embodiment, Irrigation scheduling considers entire soil profile. The soil profile comprises of solid, air and water, where water is absorbed by roots for consumptive use and empty space is filled by air. FIG.5 represents variation of water in soil profile at different time instances where (1) after irrigation, (2) during crop using soil moisture for metabolism, (3) addition of soil moisture due to rainfall, (4) soil profile reaching set allowable depletion, (5) soil profile reaching wilting point. In normal situation profile moisture varies between instance as described above (1) to (4). After stage (5) crop goes under water stress, the severity of water stress increases after wilting point after stage (5). The system and method described in this invention simulates soil profile to calculate the available soil moisture.

The amount of water required to fill the air space is calculated by soil water balance approach. The flowchart depicts Irrigation scheduling parameters such as irrigation interval, Depth of irrigation, Percent allowable depletion (based on depletion coefficient). The output of ensemble-based soil moisture estimation is used as input for irrigation scheduling. The profile soil moisture is calculated at daily interval and irrigation event is triggered based on the condition available profile moisture is less than percent allowable depletion. Once a day is identified as irrigation day the parameters specific to field and irrigation system are used and volume of water required, and time of irrigation is calculated.

***Example irrigation system parameters***

Drip - Emitter spacing, lateral spacing, Emitter discharge, weighted fraction (e.g., assumed to be 0.4 as default), System efficiency (e.g., assumed to be 90 as default).

**Drip:** No of Emitters N = (Drip Spacing * Lateral Spacing)/Plot Area

Sprinkler or micro Sprinkler - Sprinkler spacing, lateral spacing, Nozzle discharge, System efficiency (e.g., assumed to be 70 or 75 %), weighted fraction (e.g., assumed to be 0.7 or 0.8 or 1 as options).

**Sprinkler or micro Sprinkler** : No of Emitters N = (Sprinkler Spacing * Length of pipeline) or plot area.

Surface - Size of Pump (Pump Discharge), Water Conveyance Efficiency (Pipe and water channel).

**Surface:** No of Emitters N = 1 (as surface irrigation as one outlet, if required add outlets)

$$\text{Volume of Water to Irrigate } V = \text{Weighted Fraction} * \text{Plot Area} * IR$$

$$\text{Time} = V/(N * \text{Emitter Discharge})$$

Note: 1. Weighted Fraction is different for irrigation type such as Drip or Sprinkler or Surface irrigation. 2. Irrigation Requirement differs for surface conditions i.e., IR or $IR_{SEL}$ or $IR_{SCC}$.

Now step 410 of the method 400, the one or more hardware processors is configured to estimate the one or more soil properties and the one or more crop characteristics by using an inverse modelling approach, wherein the one or more soil properties includes a field capacity, and the one or more crop characteristics includes the crop coefficient.

The inverse modelling is applied for estimation of the field capacity and the crop coefficient using the soil moisture values. In forward model profile soil moisture is calculated using the soil water balance showed in Equation 9, 10 and 11.

However, the soil moisture values can be easily observed using soil moisture sensors or traditional gravimetric methods. The soil moisture storage in the soil profile and depletion coefficient are utilized to trigger an irrigation event feedback comprising a time of irrigation in the field. The process of inversion modelling where observed in the soil moisture values at multiple time periods are used to calculate the field capacity and the crop coefficient. The simplified soil water balance model for inverse model is described in Equation 15,

*RAW = Irrigation + Rainfall - Runoff - Percolation + Capillary Rise - Deep Percolation - Crop Evapotranspiration* Equation 15

The crop evapotranspiration uses energy balance method for ET and range of crop coefficient values (e.g., 0.8, 0.9, 1.0, 1.1, 1.2, etc.). The APM/RAW is compared with in-situ soil moisture values as below,

If [RAW $_{t1}$ - (Root Zone Depth x Soil Moisture) $_{t1}$] $\rightarrow$ 0 (very close to zero +or - ve)

AND [RAW $_{t2}$ - (Root Zone Depth x Soil Moisture) $_{t2}$] $\rightarrow$ 0 (very close to zero +or - ve)

Then, the value of field capacity and the crop coefficient is selected. The entire assessment is performed for the vertical zone (Homogeneous portion of the field or region of interest). The values of the field capacity and the crop coefficient are then used for daily soil water balance. The steps of inverse modeling includes below in Table 1,

Table 1 - Inverse modelling

| |
|---|
| Calculate the effective rainfall, the runoff for day 1 and 1+n |
| Calculate the ET using PM for day 1 and 1+n (using different values of crop coefficient) |
| Calculate the percolation, the deep percolation, the capillary rise for day 1 and 1+n |
| Calculate the soil moisture available in the soil profile using the soil water balance for day 1 and 1+n (using different values of field capacity) |
| Collect in-situ observation of soil moisture (sensor or gravimetric method) |
| Compare the estimated soil moisture with in-situ soil moisture and identify the closest match for day 1 and 1+n |
| Select the field capacity and the crop coefficient for the close match of soil moisture. |
| Utilizing the forward model with the selected field capacity and crop coefficient and continue the soil water balance. |

At step 412 of the method 400, the one or more hardware processors is configured to estimate an ensemble-based field soil moisture using the soil water balance, and a soil moisture estimated using the IoT sensor data and a soil moisture estimated using a synthetic aperture radar satellite data.

The field soil moisture is a filtering technique estimated using the soil moisture observed using the IoT sensor data, the soil moisture observed using the synthetic aperture radar satellite data, and the soil moisture sensor as described in Equation 1,

$$Field\ soil\ moisture = f\{soil\ moisture_{ET},\ Soil\ moisture_{SAR}\ ,Soil\ moisture_{sensor}\}$$

------------Equation 16

Referring now FIG.6, the field soil moisture is estimated using a horizontal and vertical soil profile which is a function of a first set of factors and a second set of factors. The first set of factors includes the effective rainfall, an irrigation, capillary rise, the runoff, the percolation, the deep percolation, and the evapotranspiration. The second set of factors includes the runoff, the percolation is a component of the effective rainfall, and the deep percolation is a component of the vertical soil water movement. the soil moisture is estimated for a horizontal soil profile and a vertical soil profile.

At step 414 of the method 400, the one or more hardware processors is configured to estimate crop water requirement of the field using (i) the field soil moisture and (ii) a plurality of irrigation scheduling parameters comprising an irrigation interval, a depth of irrigation, a percent allowable depletion based on depletion coefficient. The crop water requirement of the field is estimated using the irrigation data, the effective rainfall information, the runoff, a percolation, capillary rise, deep percolation, and the reference crop evapotranspiration ($ET_o$).

FIG.5 depicts variation of air and water in soil profile at various time steps using the system of FIG.2, in accordance with some embodiments of the present disclosure, in accordance with some embodiments of the present disclosure.

FIG.6 depicts soil water balance using the system of FIG.2, in accordance with some embodiments of the present disclosure, in accordance with some embodiments of the present disclosure.

The written description describes the subject matter herein to enable any person skilled in the art to make and use the embodiments.

The embodiments of present disclosure herein addresses unresolved problem of water requirement. The embodiment, thus provides system and method for estimating crop water requirement using multi-sensor data fusion. Moreover, the embodiments herein further estimates crop water requirement for scheduling irrigation or horticulture activity. The method includes observations from agro-meteorological stations, field level sensors, weather forecast and satellite based earth observations in multi-spectral and synthetic aperture radar observations.

It is to be understood that the scope of the protection is extended to such a program and in addition to a computer-

readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

**1.** A processor-implemented method (400) for estimating crop water requirement, the method comprising:

receiving (402) from a field via one or more hardware processors (104) a set of inputs comprising a IoT sensor data, one or more crop characteristics, one or more agro-meteorological weather data of the field, one or more satellite earth observation data covering the field, one or more soil properties of the field, a historical irrigation data, and a weather forecast data,

wherein the one or more agro-meteorological weather data includes a temperature, a relative humidity, a wind speed, a wind direction, an effective rainfall information, and a solar radiation information,
wherein the IoT sensor data includes a soil moisture, a soil temperature, and an air temperature;

estimating (404) using the set of inputs via the one or more hardware processors (104) (i) a reference crop evapotranspiration (ET) using the one or more agro-meteorological weather data and the IoT sensor data (ii) a crop ET under standard conditions ($ET_c$) using a crop coefficient (iii) a crop ET under non-standard conditions ($ET_{pm}$) using the crop coefficient and a soil factor (iv) a remote sensing based evapotranspiration ($ET_{rs}$) using the one or more satellite earth observation data covering the field and (v) a field evapotranspiration ET ($ET_{field}$), wherein the crop ET under the non-standard conditions is grown under management and environmental conditions that differ from the standard conditions, wherein when cultivating crops in fields deviate from $ET_c$ due to non-optimal conditions including presence of pests and diseases, soil salinity, low soil fertility, water shortage or waterlogging, then the ET rate is reduced below $ET_c$ and extent of biotic stress due to pest or disease attack is calculated using a field level smartphone camera images and abiotic stress due to temperature, moisture is calculated using remote sensing-based vegetation indices, wherein the remote sensing-based ET *(ETrs)* is estimated using one or more satellite earth data comprising of multi-spectral and thermal infra-red region;

estimating (406) a runoff via the one or more hardware processors (104) using a curve number method based on the one or more agro-meteorological weather, and the one or more satellite earth observation data covering the field;
calculating (408) a soil water balance of the field via the one or more hardware processors (104) using the field level evapotranspiration ($ET_{field}$), the runoff, the effective rainfall information, the IoT sensor data, the one or more crop characteristics and the one or more soil properties;
estimating (410) via the one or more hardware processors (104) the one or more soil properties and the one or more crop characteristics by using an inverse modelling approach, wherein the one or more soil properties includes a field capacity, and the one or more crop characteristics includes the crop coefficient;
estimating (412) an ensemble-based field soil moisture via the one or more hardware processors (104) using the soil water balance, and a soil moisture estimated using the IoT sensor data and a soil moisture estimated using a synthetic aperture radar satellite data, wherein an ensemble based field soil moisture estimator dynamically adjusts one or more parameters used for crop water requirement estimation; and
estimating (414) via the one or more hardware processors (104) crop water requirement of the field using (i) the field soil moisture and (ii) a plurality of irrigation scheduling parameters comprising an irrigation interval, a depth of irrigation, a percent allowable depletion based on depletion coefficient.

**2.** The processor-implemented method (400) as claimed in claim 1, wherein the crop water requirement of the field is estimated using the irrigation data, the effective rainfall information, the runoff, a percolation, capillary rise, deep percolation, and the reference crop evapotranspiration ($ET_o$).

**3.** The processor-implemented method (400) as claimed in claim 1, wherein the field soil moisture value is estimated using a process-based soil moisture sensor, the soil moisture observed using the synthetic aperture radar satellite data, and the soil moisture observed using the IoT sensor data.

**4.** The processor-implemented method (400) as claimed in claim 3, wherein a filtering technique is applied over the field soil moisture value to correct forthcoming soil field moisture values avoiding drastic increase or decrease of the soil moisture values, and wherein the soil moisture is estimated for a horizontal soil profile and a vertical soil profile.

**5.** The processor-implemented method (400) as claimed in claim 1, wherein the inverse modelling is applied for estimation of the field capacity and the crop coefficient of the field using the field soil moisture values, wherein the soil moisture storage in the soil profile and depletion coefficient are utilized to trigger an irrigation event feedback comprising a time of irrigation in the field, and wherein the crop water requirement for consecutive day is estimated based on the irrigation event feedback.

**6.** A system (100), for estimating crop water requirement comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive from a field a set of inputs comprising a IoT sensor data, one or more crop characteristics, one or more agro-meteorological weather data of the field, one or more satellite earth observation data covering the field, one or more soil properties of the field, a historical irrigation data, and a weather forecast data,

wherein the one or more agro-meteorological weather data includes a temperature, a relative humidity, a wind speed, a wind direction, an effective rainfall information, and a solar radiation information,
wherein the IoT sensor data includes a soil moisture, a soil temperature, and an air temperature;

estimate using the set of inputs (i) a reference crop evapotranspiration (ET) using the one or more agro-meteorological weather data and the IoT sensor data (ii) a crop ET under standard conditions ($ET_c$) using a crop coefficient (iii) a crop ET under non-standard conditions ($ET_{pm}$) using the crop coefficient and a soil factor (iv) a remote sensing based evapotranspiration ($ET_{rs}$) using the one or more satellite earth observation data covering the field and (v) a field evapotranspiration ET ($ET_{field}$), wherein the crop ET under the non-standard conditions is grown under management and environmental conditions that differ from the standard conditions, wherein when cultivating crops in fields deviate from $ET_c$ due to non-optimal conditions including presence of pests and diseases, soil salinity, low soil fertility, water shortage or waterlogging, then the ET rate

is reduced below $ET_c$ and extent of biotic stress due to pest or disease attack is calculated using a field level smartphone camera images and abiotic stress due to temperature, moisture is calculated using remote sensing-based vegetation indices, wherein the remote sensing-based ET *(ETrs)* is estimated using one or more satellite earth data comprising of multi-spectral and thermal infra-red region;

estimate a runoff using a curve number method based on the one or more agro-meteorological weather, and the one or more satellite earth observation data covering the field;

calculate a soil water balance of the field using the field level evapotranspiration ($ET_{field}$), the runoff, the effective rainfall information, the IoT sensor data, the one or more crop characteristics and the one or more soil properties;

estimate the one or more soil properties and the one or more crop characteristics by using an inverse modelling approach, wherein the one or more soil properties includes a field capacity, and the one or more crop characteristics includes the crop coefficient;

estimate an ensemble-based field soil moisture using the soil water balance, and a soil moisture estimated using the IoT sensor data and a soil moisture estimated using a synthetic aperture radar satellite data, wherein an ensemble based field soil moisture estimator dynamically adjusts one or more parameters used for crop water requirement estimation; and

estimate crop water requirement of the field using (i) the field soil moisture and (ii) a plurality of irrigation scheduling parameters comprising an irrigation interval, a depth of irrigation, a percent allowable depletion based on depletion coefficient.

**7.** The system (100) as claimed in claim 6, wherein the crop water requirement of the field is estimated using the irrigation data, the effective rainfall information, the runoff, a percolation, capillary rise, deep percolation, and the reference crop evapotranspiration ($ET_o$).

**8.** The system (100) as claimed in claim 6, wherein the field soil moisture value is estimated using a process-base soil moisture sensor, the soil moisture observed using the synthetic aperture radar satellite data, and the soil moisture observed using the IoT sensor data.

**9.** The system (100) as claimed in claim 8, wherein a filtering technique is applied over the field soil moisture value to correct forthcoming soil field moisture values avoiding drastic increase or decrease of the soil moisture values, and wherein the soil moisture is estimated for a horizontal soil profile and a vertical soil profile.

**10.** The system (100) as claimed in claim 6, wherein the inverse modelling is applied for estimation of the field capacity and the crop coefficient of the field using the field soil moisture values, wherein the soil moisture storage in the soil profile and depletion coefficient are utilized to trigger an irrigation event feedback comprising a time of irrigation in the field, and wherein the crop water requirement for consecutive day is estimated based on the irrigation event feedback.

**11.** One or more non-transitory machine-readable information storage mediums comprising one or more instructions which when executed by one or more hardware processors cause:

receive from a field a set of inputs comprising a IoT sensor data, one or more crop characteristics, one or more agro-meteorological weather data of the field, one or more satellite earth observation data covering the field, one or more soil properties of the field, a historical irrigation data, and a weather forecast data,

wherein the one or more agro-meteorological weather data includes a temperature, a relative humidity, a wind speed, a wind direction, an effective rainfall information, and a solar radiation information,

wherein the IoT sensor data includes a soil moisture, a soil temperature, and an air temperature;

estimate using the set of inputs (i) a reference crop evapotranspiration (ET) using the one or more agro-meteorological weather data and the IoT sensor data (ii) a crop ET under standard conditions ($ET_c$) using a crop coefficient (iii) a crop ET under non-standard conditions ($ET_{pm}$) using the crop coefficient and a soil factor (iv) a remote sensing based evapotranspiration ($ET_{rs}$) using the one or more satellite earth observation data covering the field and (v) a field evapotranspiration ET ($ET_{field}$), wherein the crop ET under the non-standard conditions is grown under management and environmental conditions that differ from the standard conditions, wherein when cultivating crops in fields deviate from $ET_c$ due to non-optimal conditions including presence of pests and diseases, soil salinity, low soil fertility, water shortage or waterlogging, then the ET rate is reduced below $ET_c$ and extent of biotic stress due to pest or disease attack is calculated using a field level smartphone camera images and abiotic stress due to temperature, moisture is calculated using remote sensing-based vegetation indices, wherein

the remote sensing-based ET (*ETrs*) is estimated using one or more satellite earth data comprising of multispectral and thermal infra-red region;
estimate a runoff using a curve number method based on the one or more agro-meteorological weather, and the one or more satellite earth observation data covering the field;
calculate a soil water balance of the field using the field level evapotranspiration ($ET_{field}$), the runoff, the effective rainfall information, the IoT sensor data, the one or more crop characteristics and the one or more soil properties;
estimate the one or more soil properties and the one or more crop characteristics by using an inverse modelling approach, wherein the one or more soil properties includes a field capacity, and the one or more crop characteristics includes the crop coefficient;
estimate an ensemble-based field soil moisture using the soil water balance, and a soil moisture estimated using the IoT sensor data and a soil moisture estimated using a synthetic aperture radar satellite data, wherein an ensemble based field soil moisture estimator dynamically adjusts one or more parameters used for crop water requirement estimation; and
estimate crop water requirement of the field using (i) the field soil moisture and (ii) a plurality of irrigation scheduling parameters comprising an irrigation interval, a depth of irrigation, a percent allowable depletion based on depletion coefficient.

**12.** The one or more non-transitory machine-readable information storage mediums of claim 11, wherein the crop water requirement of the field is estimated using the irrigation data, the effective rainfall information, the runoff, a percolation, capillary rise, deep percolation, and the reference crop evapotranspiration ($ET_o$).

**13.** The one or more non-transitory machine-readable information storage mediums of claim 11, wherein the field soil moisture value is estimated using a process-base soil moisture sensor, the soil moisture observed using the synthetic aperture radar satellite data, and the soil moisture observed using the IoT sensor data.

**14.** The one or more non-transitory machine-readable information storage mediums of claim 13, wherein a filtering technique is applied over the field soil moisture value to correct forthcoming soil field moisture values avoiding drastic increase or decrease of the soil moisture values, and wherein the soil moisture is estimated for a horizontal soil profile and a vertical soil profile.

**15.** The one or more non-transitory machine-readable information storage mediums of claim 11, wherein the inverse modelling is applied for estimation of the field capacity and the crop coefficient of the field using the field soil moisture values, wherein the soil moisture storage in the soil profile and depletion coefficient are utilized to trigger an irrigation event feedback comprising a time of irrigation in the field, and wherein the crop water requirement for consecutive day is estimated based on the irrigation event feedback.

## Patentansprüche

**1.** Prozessorimplementiertes Verfahren (400) zum Schätzen des Erntewasserbedarfs, wobei das Verfahren Folgendes umfasst:

Empfangen (402), von einem Feld über einen oder mehrere Hardwareprozessoren (104), eines Satzes von Eingaben, umfassend IoT-Sensordaten, eine oder mehrere Ernteeigenschaften, eine oder mehrere agrometeorologische Wetterdaten des Feldes, eine oder mehrere Satellitenerdbeobachtungsdaten, die das Feld abdecken, eine oder mehrere Bodeneigenschaften des Feldes, historische Bewässerungsdaten und Wettervorhersagedaten,

wobei die eine oder die mehreren agrometeorologischen Wetterdaten eine Temperatur, eine relative Luftfeuchtigkeit, eine Windgeschwindigkeit, eine Windrichtung, eine Information über den effektiven Niederschlag und eine Sonnenstrahlungsinformation beinhalten,
wobei die IoT-Sensordaten eine Bodenfeuchtigkeit, eine Bodentemperatur und eine Lufttemperatur beinhalten;

Schätzen (404), unter Verwendung des Satzes von Eingaben über den einen oder die mehreren Hardwareprozessoren (104), (i) einer Referenz-Ernteevapotranspiration (ET) unter Verwendung der einen oder der mehreren agrometeorologischen Wetterdaten und der IoT-Sensordaten, (ii) einer Ernte-ET unter Standardbedingungen ($ET_c$) unter Verwendung eines Erntekoeffizienten, (iii) einer Ernte-ET unter Nicht-Standardbedin-

gungen ($ET_{pm}$) unter Verwendung des Erntekoeffizienten und eines Bodenfaktors, (iv) einer auf Fernerfassung basierenden Evapotranspiration ($ET_{rs}$) unter Verwendung der einen oder der mehreren Satellitenerdbeobachtungsdaten, die das Feld abdecken, und (v) einer Feld-Evapotranspirations-ET ($ET_{feld}$), wobei die Ernte-ET unter den Nicht-Standardbedingungen unter Management- und Umweltbedingungen gezüchtet wird, die sich von den Standardbedingungen unterscheiden, wobei, wenn die Ernte auf Feldern aufgrund nicht optimaler Bedingungen, einschließlich des Vorhandenseins von Schädlingen und Krankheiten, des Bodensalzgehalts, der geringen Bodenfruchtbarkeit, des Wassermangels oder der Wasserverstopfung, von $ET_c$ abgewichen wird, dann die ET-Rate unter $ET_c$ reduziert wird und das Ausmaß des biotischen Stresses aufgrund von Schädling- oder Krankheitsbefall unter Verwendung von Bildern einer Smartphone-Kamera auf Feldebene und abiotischem Stress aufgrund von Temperatur berechnet wird, Feuchtigkeit unter Verwendung von auf Fernerfassung basierenden Vegetationsindizes berechnet wird, wobei die auf Fernerfassung basierende ET (*ETrs*) unter Verwendung von einer oder mehreren Satellitenerddaten geschätzt wird, die einen multispektralen und thermischen Infrarotbereich umfassen;

Schätzen (406) eines Ablaufs über den einen oder die mehreren Hardwareprozessoren (104) unter Verwendung eines Kurvenzahlverfahrens basierend auf dem einen oder den mehreren agrometeorologischen Wetterdaten und der einen oder den mehreren Satellitenerdbeobachtungsdaten, die das Feld abdecken;

Berechnen (408) einer Bodenwasserbilanz des Feldes über den einen oder die mehreren Hardwareprozessoren (104) unter Verwendung der Feldebenen-Evapotranspiration ($ET_{feld}$), des Ablaufs, der Information über den effektiven Niederschlag, der IoT-Sensordaten, der einen oder der mehreren Ernteeigenschaften und der einen oder der mehreren Bodeneigenschaften;

Schätzen (410), über den einen oder die mehreren Hardwareprozessoren (104), der einen oder der mehreren Bodeneigenschaften und der einen oder der mehreren Ernteeigenschaften unter Verwendung eines inversen Modellierungsansatzes, wobei die eine oder die mehreren Bodeneigenschaften eine Feldkapazität beinhalten und die eine oder die mehreren Ernteeigenschaften den Erntekoeffizienten beinhalten;

Schätzen (412) einer auf einem Ensemble basierenden Feldbodenfeuchtigkeit über den einen oder die mehreren Hardwareprozessoren (104) unter Verwendung der Bodenwasserbilanz und einer Bodenfeuchtigkeit, die unter Verwendung der IoT-Sensordaten geschätzt wird, und einer Bodenfeuchtigkeit, die unter Verwendung von Radarsatellitendaten mit synthetischer Apertur geschätzt wird, wobei ein auf einem Ensemble basierender Feldbodenfeuchtigkeitsschätzer einen oder mehrere Parameter, die zur Schätzung des Erntewasserbedarfs verwendet werden, dynamisch anpasst; und

Schätzen (414), über den einen oder die mehreren Hardwareprozessoren (104), des Erntewasserbedarfs des Feldes unter Verwendung (i) der Feldbodenfeuchtigkeit und (ii) einer Mehrzahl von Bewässerungsplanungsparametern, die ein Bewässerungsintervall, eine Bewässerungstiefe, eine prozentuale zulässige Erschöpfung basierend auf dem Erschöpfungskoeffizienten umfassen.

**2.** Prozessorimplementiertes Verfahren (400) nach Anspruch 1, wobei der Erntewasserbedarf des Feldes unter Verwendung der Bewässerungsdaten, der Information über den effektiven Niederschlag, des Ablaufs, einer Perkolation, eines Kapillaranstiegs, einer tiefen Perkolation und der Referenz-Ernteevapotranspiration ($ET_o$) geschätzt wird.

**3.** Prozessorimplementiertes Verfahren (400) nach Anspruch 1, wobei der Feldbodenfeuchtigkeitswert unter Verwendung eines prozessbasierten Bodenfeuchtigkeitssensors, der Bodenfeuchtigkeit, die unter Verwendung der Radarsatellitendaten mit synthetischer Apertur beobachtet wird, und der Bodenfeuchtigkeit, die unter Verwendung der IoT-Sensordaten beobachtet wird, geschätzt wird.

**4.** Prozessorimplementiertes Verfahren (400) nach Anspruch 3, wobei eine Filtertechnik über den Feldbodenfeuchtigkeitswert angewendet wird, um bevorstehende Bodenfeldfeuchtigkeitswerte zu korrigieren, wobei eine drastische Erhöhung oder Verringerung der Bodenfeuchtigkeitswerte vermieden wird, und wobei die Bodenfeuchtigkeit für ein horizontales Bodenprofil und ein vertikales Bodenprofil geschätzt wird.

**5.** Prozessorimplementiertes Verfahren (400) nach Anspruch 1, wobei die inverse Modellierung zur Schätzung der Feldkapazität und des Erntekoeffizienten des Feldes unter Verwendung der Feldbodenfeuchtigkeitswerte angewendet wird, wobei die Bodenfeuchtigkeitsspeicherung in dem Bodenprofil und der Erschöpfungskoeffizient verwendet werden, um eine Bewässerungsereignisrückmeldung auszulösen, die eine Zeit der Bewässerung in dem Feld umfasst, und wobei der Erntewasserbedarf für einen aufeinanderfolgenden Tag basierend auf der Bewässerungsereignisrückmeldung geschätzt wird.

**6.** System (100) zum Schätzen des Erntewasserbedarfs, das Folgendes umfasst:

einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

Empfangen, von einem Feld, eines Satzes von Eingaben, umfassend IoT-Sensordaten, eine oder mehrere Ernteeigenschaften, eine oder mehrere agrometeorologische Wetterdaten des Feldes, eine oder mehrere Satellitenerdbeobachtungsdaten, die das Feld abdecken, eine oder mehrere Bodeneigenschaften des Feldes, historische Bewässerungsdaten und Wettervorhersagedaten,

wobei die eine oder die mehreren agrometeorologischen Wetterdaten eine Temperatur, eine relative Luftfeuchtigkeit, eine Windgeschwindigkeit, eine Windrichtung, eine Information über den effektiven Niederschlag und eine Sonnenstrahlungsinformation beinhalten,
wobei die IoT-Sensordaten eine Bodenfeuchtigkeit, eine Bodentemperatur und eine Lufttemperatur beinhalten;

Schätzen, unter Verwendung des Satzes von Eingaben, (i) einer Referenz-Ernteevapotranspiration (ET) unter Verwendung der einen oder der mehreren agrometeorologischen Wetterdaten und der IoT-Sensordaten, (ii) einer Ernte-ET unter Standardbedingungen ($ET_c$) unter Verwendung eines Erntekoeffizienten, (iii) einer Ernte-ET unter Nicht-Standardbedingungen ($ET_{pm}$) unter Verwendung des Erntekoeffizienten und eines Bodenfaktors, (iv) einer auf Fernerfassung basierenden Evapotranspiration ($ET_{rs}$) unter Verwendung der einen oder der mehreren Satellitenerdbeobachtungsdaten, die das Feld abdecken, und (v) einer Feld-Evapotranspirations-ET ($ET_{feld}$), wobei die Ernte-ET unter den Nicht-Standardbedingungen unter Management- und Umweltbedingungen gezüchtet wird, die sich von den Standardbedingungen unterscheiden, wobei, wenn die Ernte auf Feldern aufgrund nicht optimaler Bedingungen, einschließlich des Vorhandenseins von Schädlingen und Krankheiten, des Bodensalzgehalts, der geringen Bodenfruchtbarkeit, des Wassermangels oder der Wasserverstopfung, von $ET_c$ abgewichen wird, dann die ET-Rate unter $ET_c$ reduziert wird und das Ausmaß des biotischen Stresses aufgrund von Schädling- oder Krankheitsbefall unter Verwendung von Bildern einer Smartphone-Kamera auf Feldebene und abiotischem Stress aufgrund von Temperatur berechnet wird, Feuchtigkeit unter Verwendung von auf Fernerfassung basierenden Vegetationsindizes berechnet wird, wobei die auf Fernerfassung basierende ET ($ETrs$) unter Verwendung von einer oder mehreren Satellitenerddaten geschätzt wird, die einen multispektralen und thermischen Infrarotbereich umfassen;
Schätzen eines Ablaufs unter Verwendung eines Kurvenzahlverfahrens basierend auf dem einen oder den mehreren agrometeorologischen Wetterdaten und der einen oder den mehreren Satellitenerdbeobachtungsdaten, die das Feld abdecken;
Berechnen einer Bodenwasserbilanz des Feldes unter Verwendung der Feldebenen-Evapotranspiration ($ET_{feld}$), des Ablaufs, der Information über den effektiven Niederschlag, der IoT-Sensordaten, der einen oder der mehreren Ernteeigenschaften und der einen oder der mehreren Bodeneigenschaften;
Schätzen der einen oder der mehreren Bodeneigenschaften und der einen oder der mehreren Ernteeigenschaften unter Verwendung eines inversen Modellierungsansatzes, wobei die eine oder die mehreren Bodeneigenschaften eine Feldkapazität beinhalten und die eine oder die mehreren Ernteeigenschaften den Erntekoeffizienten beinhalten;
Schätzen einer auf einem Ensemble basierenden Feldbodenfeuchtigkeit unter Verwendung der Bodenwasserbilanz und einer Bodenfeuchtigkeit, die unter Verwendung der IoT-Sensordaten geschätzt wird, und einer Bodenfeuchtigkeit, die unter Verwendung von Radarsatellitendaten mit synthetischer Apertur geschätzt wird, wobei ein auf einem Ensemble basierender Feldbodenfeuchtigkeitsschätzer einen oder mehrere Parameter, die zur Schätzung des Erntewasserbedarfs verwendet werden, dynamisch anpasst; und
Schätzen des Erntewasserbedarfs des Feldes unter Verwendung (i) der Feldbodenfeuchtigkeit und (ii) einer Mehrzahl von Bewässerungsplanungsparametern, die ein Bewässerungsintervall, eine Bewässerungstiefe, eine prozentuale zulässige Erschöpfung basierend auf dem Erschöpfungskoeffizienten umfassen.

**7.** System (100) nach Anspruch 6, wobei der Erntewasserbedarf des Feldes unter Verwendung der Bewässerungsdaten, der Information über den effektiven Niederschlag, des Ablaufs, einer Perkolation, eines Kapillaranstiegs, einer tiefen Perkolation und der Referenz-Ernteevapotranspiration ($ET_o$) geschätzt wird.

**8.** System (100) nach Anspruch 6, wobei der Feldbodenfeuchtigkeitswert unter Verwendung eines prozessbasierten Bodenfeuchtigkeitssensors, der Bodenfeuchtigkeit, die unter Verwendung der Radarsatellitendaten mit synthetischer Apertur beobachtet wird, und der Bodenfeuchtigkeit, die unter Verwendung der IoT-Sensordaten beobachtet wird, geschätzt wird.

**9.** System (100) nach Anspruch 8, wobei eine Filtertechnik über den Feldbodenfeuchtigkeitswert angewendet wird, um bevorstehende Bodenfeldfeuchtigkeitswerte zu korrigieren, wobei eine drastische Erhöhung oder Verringerung der Bodenfeuchtigkeitswerte vermieden wird, und wobei die Bodenfeuchtigkeit für ein horizontales Bodenprofil und ein vertikales Bodenprofil geschätzt wird.

**10.** System (100) nach Anspruch 6, wobei die inverse Modellierung zur Schätzung der Feldkapazität und des Erntekoeffizienten des Feldes unter Verwendung der Feldbodenfeuchtigkeitswerte angewendet wird, wobei die Bodenfeuchtigkeitsspeicherung in dem Bodenprofil und der Erschöpfungskoeffizient verwendet werden, um eine Bewässerungsereignisrückmeldung auszulösen, die eine Zeit der Bewässerung in dem Feld umfasst, und wobei der Erntewasserbedarf für einen aufeinanderfolgenden Tag basierend auf der Bewässerungsereignisrückmeldung geschätzt wird.

**11.** Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, bewirken, dass:

von einem Feld ein Satz von Eingaben empfangen wird, umfassend IoT-Sensordaten, eine oder mehrere Ernteeigenschaften, eine oder mehrere agrometeorologische Wetterdaten des Feldes, eine oder mehrere Satellitenerdbeobachtungsdaten, die das Feld abdecken, eine oder mehrere Bodeneigenschaften des Feldes, historische Bewässerungsdaten und Wettervorhersagedaten,

wobei die eine oder die mehreren agrometeorologischen Wetterdaten eine Temperatur, eine relative Luftfeuchtigkeit, eine Windgeschwindigkeit, eine Windrichtung, eine Information über den effektiven Niederschlag und eine Sonnenstrahlungsinformation beinhalten,
wobei die IoT-Sensordaten eine Bodenfeuchtigkeit, eine Bodentemperatur und eine Lufttemperatur beinhalten;

unter Verwendung des Satzes von Eingaben (i) eine Referenz-Ernteevapotranspiration (ET) unter Verwendung der einen oder der mehreren agrometeorologischen Wetterdaten und der IoT-Sensordaten, (ii) eine Ernte-ET unter Standardbedingungen ($ET_c$) unter Verwendung eines Erntekoeffizienten, (iii) eine Ernte-ET unter Nicht-Standardbedingungen ($ET_{pm}$) unter Verwendung des Erntekoeffizienten und eines Bodenfaktors, (iv) eine auf Fernerfassung basierende Evapotranspiration ($ET_{rs}$) unter Verwendung der einen oder der mehreren Satellitenerdbeobachtungsdaten, die das Feld abdecken, und (v) eine Feld-Evapotranspirations-ET ($ET_{feld}$), wobei die Ernte-ET unter den Nicht-Standardbedingungen unter Management- und Umweltbedingungen gezüchtet wird, die sich von den Standardbedingungen unterscheiden, wobei, wenn die Ernte auf Feldern aufgrund nicht optimaler Bedingungen, einschließlich des Vorhandenseins von Schädlingen und Krankheiten, des Bodensalzgehalts, der geringen Bodenfruchtbarkeit, des Wassermangels oder der Wasserverstopfung, von $ET_c$ abgewichen wird, dann die ET-Rate unter $ET_c$ reduziert wird und das Ausmaß des biotischen Stresses aufgrund von Schädling- oder Krankheitsbefall unter Verwendung von Bildern einer Smartphone-Kamera auf Feldebene und abiotischem Stress aufgrund von Temperatur berechnet wird, Feuchtigkeit unter Verwendung von auf Fernerfassung basierenden Vegetationsindizes berechnet wird, wobei die auf Fernerfassung basierende ET (*ETrs*) unter Verwendung von einer oder mehreren Satellitenerddaten geschätzt wird, die einen multispektralen und thermischen Infrarotbereich umfassen;
einen Ablauf unter Verwendung eines Kurvenzahlverfahrens basierend auf dem einen oder den mehreren agrometeorologischen Wetterdaten und der einen oder den mehreren Satellitenerdbeobachtungsdaten, die das Feld abdecken, schätzt;
eine Bodenwasserbilanz des Feldes unter Verwendung der Feldebenen-Evapotranspiration ($ET_{feld}$), des Ablaufs, der Information über den effektiven Niederschlag, der IoT-Sensordaten, der einen oder der mehreren Ernteeigenschaften und der einen oder der mehreren Bodeneigenschaften berechnet;
die eine oder die mehreren Bodeneigenschaften und die eine oder die mehreren Ernteeigenschaften unter Verwendung eines inversen Modellierungsansatzes schätzt, wobei die eine oder die mehreren Bodeneigenschaften eine Feldkapazität beinhalten und die eine oder die mehreren Ernteeigenschaften den Erntekoeffizienten beinhalten;
eine auf einem Ensemble basierende Feldbodenfeuchtigkeit unter Verwendung der Bodenwasserbilanz und

einer Bodenfeuchtigkeit, die unter Verwendung der IoT-Sensordaten geschätzt wird, und einer Bodenfeuchtigkeit, die unter Verwendung von Radarsatellitendaten mit synthetischer Apertur geschätzt wird, schätzt, wobei ein auf einem Ensemble basierender Feldbodenfeuchtigkeitsschätzer einen oder mehrere Parameter, die zur Schätzung des Erntewasserbedarfs verwendet werden, dynamisch anpasst; und
den Erntewasserbedarf des Feldes unter Verwendung (i) der Feldbodenfeuchtigkeit und (ii) einer Mehrzahl von Bewässerungsplanungsparametern, die ein Bewässerungsintervall, eine Bewässerungstiefe, eine prozentuale zulässige Erschöpfung basierend auf dem Erschöpfungskoeffizienten umfassen, schätzt.

**12.** Ein oder mehrere nichtflüchtige maschinenlesbare Informationsspeichermedien nach Anspruch 11, wobei der Erntewasserbedarf des Feldes unter Verwendung der Bewässerungsdaten, der Information über den effektiven Niederschlag, des Ablaufs, einer Perkolation, eines Kapillaranstiegs, einer tiefen Perkolation und der Referenz-Ernteevapotranspiration ($ET_o$) geschätzt wird.

**13.** Ein oder mehrere nichtflüchtige maschinenlesbare Informationsspeichermedien nach Anspruch 11, wobei der Feldbodenfeuchtigkeitswert unter Verwendung eines prozessbasierten Bodenfeuchtigkeitssensors, der Bodenfeuchtigkeit, die unter Verwendung der Radarsatellitendaten mit synthetischer Apertur beobachtet wird, und der Bodenfeuchtigkeit, die unter Verwendung der IoT-Sensordaten beobachtet wird, geschätzt wird.

**14.** Ein oder mehrere nichtflüchtige maschinenlesbare Informationsspeichermedien nach Anspruch 13, wobei eine Filtertechnik über den Feldbodenfeuchtigkeitswert angewendet wird, um bevorstehende Bodenfeldfeuchtigkeitswerte zu korrigieren, wobei eine drastische Erhöhung oder Verringerung der Bodenfeuchtigkeitswerte vermieden wird, und wobei die Bodenfeuchtigkeit für ein horizontales Bodenprofil und ein vertikales Bodenprofil geschätzt wird.

**15.** Ein oder mehrere nichtflüchtige maschinenlesbare Informationsspeichermedien nach Anspruch 11, wobei die inverse Modellierung zur Schätzung der Feldkapazität und des Erntekoeffizienten des Feldes unter Verwendung der Feldbodenfeuchtigkeitswerte angewendet wird, wobei die Bodenfeuchtigkeitsspeicherung in dem Bodenprofil und der Erschöpfungskoeffizient verwendet werden, um eine Bewässerungsereignisrückmeldung auszulösen, die eine Zeit der Bewässerung in dem Feld umfasst, und wobei der Erntewasserbedarf für einen aufeinanderfolgenden Tag basierend auf der Bewässerungsereignisrückmeldung geschätzt wird.

## Revendications

**1.** Procédé mis en œuvre par processeur (400) pour estimer un besoin en eau de la culture, le procédé comprenant :

la réception (402) à partir d'un champ via un ou plusieurs processeurs matériels (104) d'un ensemble d'entrées comprenant des données de capteur IoT, une ou plusieurs caractéristiques de la culture, une ou plusieurs données agrométéorologiques du champ, une ou plusieurs données satellitaires d'observation de la Terre couvrant le champ, une ou plusieurs propriétés de sol du champ, des données d'irrigation historiques, et des données de prévisions météorologiques,

dans lequel les une ou plusieurs données agrométéorologiques incluent une température, une humidité relative, une vitesse du vent, une direction du vent, des informations de précipitations effectives, et des informations de rayonnement solaire,
dans lequel les données de capteur IoT incluent une humidité du sol, une température du sol, et une température de l'air ;

l'estimation (404) à l'aide de l'ensemble d'entrées via les un ou plusieurs processeurs matériels (104) (i) d'une évapotranspiration (ET) de référence à l'aide des une ou plusieurs données agrométéorologiques et des données de capteur IoT (ii) d'une ET de la culture dans des conditions standard ($ET_c$) à l'aide d'un coefficient cultural (iii) d'une ET de la culture dans des conditions non standard ($ET_{pm}$) à l'aide du coefficient cultural et d'un facteur de sol (iv) d'une évapotranspiration basée sur la télédétection ($ET_{rs}$) à l'aide des une ou plusieurs données satellitaires d'observation de la Terre couvrant le champ et (v) d'une ET d'évapotranspiration de champ ($ET_{champ}$), dans lequel l'ET de la culture dans les conditions non standard est cultivée dans des conditions de gestion et environnementales qui diffèrent des conditions standard, dans lequel lorsque les cultures dans des champs s'écartent de l'$ET_c$ en raison de conditions non optimales incluant la présence de parasites et de maladies, la salinité du sol, la faible fertilité du sol, la pénurie d'eau ou l'engorgement du sol, alors le taux d'ET est réduit en dessous de l'$ET_c$ et l'étendue du stress biotique dû à une attaque par un parasite ou une maladie est calculée à

l'aide d'images de caméra de smartphone de niveau de champ et d'un stress abiotique dû à la température, l'humidité est calculée à l'aide d'indices de végétation basés sur la télédétection, dans lequel l'ET basée sur la télédétection (*ETrs*) est estimée à l'aide d'une ou plusieurs données satellitaires comprenant des bandes multispectrales et infrarouges thermiques ;
l'estimation (406) d'un ruissellement via les un ou plusieurs processeurs matériels (104) à l'aide d'une méthode du numéro de courbe basée sur les une ou plusieurs données agrométéorologiques, et les une ou plusieurs données satellitaires d'observation de la Terre couvrant le champ ;
le calcul (408) d'un bilan hydrique du sol du champ via les un ou plusieurs processeurs matériels (104) à l'aide de l'évapotranspiration de niveau de champ ($ET_{champ}$), du ruissellement, des informations de précipitations effectives, des données de capteur IoT, des une ou plusieurs caractéristiques de la culture et des une ou plusieurs propriétés de sol ;
l'estimation (410) via les un ou plusieurs processeurs matériels (104) des une ou plusieurs propriétés de sol et des une ou plusieurs caractéristiques de la culture à l'aide d'une approche de modélisation inverse, dans lequel les une ou plusieurs propriétés de sol incluent une capacité au champ, et les une ou plusieurs caractéristiques de la culture incluent le coefficient cultural ;
l'estimation (412) d'ensemble de l'humidité du sol au champ via les un ou plusieurs processeurs matériels (104) à l'aide du bilan hydrique du sol, et d'une humidité de sol estimée à l'aide des données de capteur IoT et d'une humidité de sol estimée à l'aide de données satellitaires radar à synthèse d'ouverture (SAR), dans lequel un estimateur d'ensemble de l'humidité du sol au champ ajuste dynamiquement un ou plusieurs paramètres utilisés pour une estimation du besoin en eau de la culture ; et
l'estimation (414) via les un ou plusieurs processeurs matériels (104) d'un besoin en eau de la culture du champ à l'aide (i) de l'humidité du sol au champ et (ii) d'une pluralité de paramètres de programmation d'irrigation comprenant un intervalle d'irrigation, une dose d'irrigation, un pourcentage d'épuisement admissible basé sur un coefficient d'épuisement.

**2.** Procédé mis en œuvre par processeur (400) selon la revendication 1, dans lequel le besoin en eau de la culture du champ est estimé à l'aide des données d'irrigation, des informations de précipitations effectives, du ruissellement, d'une percolation, d'une remontée capillaire, d'une percolation profonde, et de l'évapotranspiration de référence ($ET_o$).

**3.** Procédé mis en œuvre par processeur (400) selon la revendication 1, dans lequel la valeur d'humidité du sol au champ est estimée à l'aide d'un capteur d'humidité de sol basé sur un processus, de l'humidité de sol observée à l'aide des données satellitaires radar à synthèse d'ouverture (SAR), et de l'humidité de sol observée à l'aide des données de capteur IoT.

**4.** Procédé mis en œuvre par processeur (400) selon la revendication 3, dans lequel une technique de filtrage est appliquée sur la valeur d'humidité du sol au champ pour corriger des valeurs à venir d'humidité du sol au champ évitant une augmentation ou une diminution drastique des valeurs d'humidité de sol, et lequel l'humidité de sol est estimée pour un profil de sol horizontal et un profil de sol vertical.

**5.** Procédé mis en œuvre par processeur (400) selon la revendication 1, dans lequel la modélisation inverse est appliquée pour une estimation de la capacité au champ et du coefficient cultural du champ à l'aide des valeurs d'humidité du sol au champ, dans lequel le stockage d'humidité de sol dans le profil de sol et le coefficient d'épuisement sont utilisés pour déclencher une rétroaction d'événement d'irrigation comprenant un temps d'irrigation dans le champ, et dans lequel le besoin en eau de la culture pour une journée consécutive est estimé sur la base de la rétroaction d'événement d'irrigation.

**6.** Système (100), pour estimer un besoin en eau de la culture comprenant :

une mémoire (102) stockant des instructions ;
une ou plusieurs interfaces de communication (106) ; et
un ou plusieurs processeurs matériels (104) couplés à la mémoire (102) via les une ou plusieurs interfaces de communication (106), dans lequel les un ou
plusieurs processeurs matériels (104) sont configurés par les instructions pour

recevoir à partir d'un champ un ensemble d'entrées comprenant des données de capteur IoT, une ou plusieurs caractéristiques de la culture, une ou plusieurs données agrométéorologiques du champ, une ou plusieurs données satellitaires d'observation de la Terre couvrant le champ, une ou plusieurs propriétés de

sol du champ, des données d'irrigation historiques, et des données de prévisions météorologiques,

dans lequel les une ou plusieurs données agrométéorologiques incluent une température, une humidité relative, une vitesse du vent, une direction du vent, des informations de précipitations effectives, et des informations de rayonnement solaire,
dans lequel les données de capteur IoT incluent une humidité du sol, une température du sol, et une température de l'air ;

estimer à l'aide de l'ensemble d'entrées (i) une évapotranspiration (ET) de référence à l'aide des une ou plusieurs données agrométéorologiques et des données de capteur IoT (ii) une ET de la culture dans des conditions standard ($ET_c$) à l'aide d'un coefficient cultural (iii) d'une ET de la culture dans des conditions non standard ($ET_{pm}$) à l'aide du coefficient cultural et d'un facteur de sol (iv) d'une évapotranspiration basée sur la télédétection ($ET_{rs}$) à l'aide des une ou plusieurs données satellitaires d'observation de la Terre couvrant le champ et (v) d'une ET d'évapotranspiration de champ ($ET_{champ}$), dans lequel l'ET de la culture dans les conditions non standard est cultivée dans des conditions de gestion et environnementales qui diffèrent des conditions standard, dans lequel lorsque les cultures dans des champs s'écartent de l'$ET_c$ en raison de conditions non optimales incluant la présence de parasites et de maladies, la salinité du sol, la faible fertilité du sol, la pénurie d'eau ou l'engorgement du sol, alors le taux d'ET est réduit en dessous de l'$ET_c$ et l'étendue du stress biotique dû à une attaque par un parasite ou une maladie est calculée à l'aide d'images de caméra de smartphone de niveau de champ et d'un stress abiotique dû à la température, l'humidité est calculée à l'aide d'indices de végétation basés sur la télédétection, dans lequel l'ET basée sur la télédétection (*ETrs*) est estimée à l'aide d'une ou plusieurs données satellitaires comprenant des bandes multispectrales et infrarouges thermiques ;
estimer un ruissellement à l'aide d'une méthode du numéro de courbe basée sur les une ou plusieurs données agrométéorologiques, et les une ou plusieurs données satellitaires d'observation de la Terre couvrant le champ ;
calculer un bilan hydrique du sol du champ à l'aide de l'évapotranspiration de niveau de champ ($ET_{champ}$), du ruissellement, des informations de précipitations effectives, des données de capteur IoT, des une ou plusieurs caractéristiques de la culture et des une ou plusieurs propriétés de sol ;
estimer les une ou plusieurs propriétés de sol et les une ou plusieurs caractéristiques de la culture à l'aide d'une approche de modélisation inverse, dans lequel les une ou plusieurs propriétés de sol incluent une capacité au champ, et les une ou plusieurs caractéristiques de la culture incluent le coefficient cultural ;
estimer, selon une approche d'ensemble, l'humidité du sol au champ à l'aide du bilan hydrique du sol, et une humidité de sol estimée à l'aide des données de capteur IoT et une humidité de sol estimée à l'aide de données satellitaires radar à synthèse d'ouverture (SAR), dans lequel un estimateur d'ensemble de l'humidité du sol au champ ajuste dynamiquement un ou plusieurs paramètres utilisés pour une estimation du besoin en eau de la culture ; et
estimer un besoin en eau de la culture du champ à l'aide (i) de l'humidité du sol au champ et (ii) d'une pluralité de paramètres de programmation d'irrigation comprenant un intervalle d'irrigation, une dose d'irrigation, un pourcentage d'épuisement admissible basé sur un coefficient d'épuisement.

**7.** Système (100) selon la revendication 6, dans lequel le besoin en eau de la culture du champ est estimé à l'aide des données d'irrigation, des informations de précipitations effectives, du ruissellement, d'une percolation, d'une remontée capillaire, d'une percolation profonde, et de l'évapotranspiration de référence ($ET_o$).

**8.** Système (100) selon la revendication 6, dans lequel la valeur d'humidité du sol au champ est estimée à l'aide d'un capteur d'humidité de sol basé sur un processus, de l'humidité de sol observée à l'aide des données satellitaires radar à synthèse d'ouverture (SAR), et de l'humidité de sol observée à l'aide des données de capteur IoT.

**9.** Système (100) selon la revendication 8, dans lequel une technique de filtrage est appliquée sur la valeur d'humidité du sol au champ pour corriger des valeurs à venir d'humidité du sol au champ évitant une augmentation ou une diminution drastique des valeurs d'humidité de sol, et dans lequel l'humidité de sol est estimée pour un profil de sol horizontal et un profil de sol vertical.

**10.** Système (100) selon la revendication 6, dans lequel la modélisation inverse est appliquée pour une estimation de la capacité au champ et du coefficient cultural du champ à l'aide des valeurs d'humidité du sol au champ, dans lequel le stockage d'humidité de sol dans le profil de sol et le coefficient d'épuisement sont utilisés pour déclencher une rétroaction d'événement d'irrigation comprenant un temps d'irrigation dans le champ, et dans lequel le besoin en eau

de la culture pour une journée consécutive est estimé sur la base de la rétroaction d'événement d'irrigation.

**11.** Un ou plusieurs supports de stockage d'informations lisibles par machine non transitoires comprenant une ou plusieurs instructions qui, lorsqu'elles sont exécutées par un ou plusieurs processeurs matériels, amènent :

à recevoir à partir d'un champ un ensemble d'entrées comprenant des données de capteur IoT, une ou plusieurs caractéristiques de la culture, une ou plusieurs données agrométéorologiques du champ, une ou plusieurs données satellitaires d'observation de la Terre couvrant le champ, une ou plusieurs propriétés de sol du champ, des données d'irrigation historiques, et des données de prévisions météorologiques,

dans lequel les une ou plusieurs données agrométéorologiques incluent une température, une humidité relative, une vitesse du vent, une direction du vent, des informations de précipitations effectives, et des informations de rayonnement solaire,
dans lequel les données de capteur IoT incluent une humidité du sol, une température du sol, et une température de l'air ;

à estimer à l'aide de l'ensemble d'entrées (i) une évapotranspiration (ET) de référence à l'aide des une ou plusieurs données agrométéorologiques et des données de capteur IoT (ii) une ET de la culture dans des conditions standard ($ET_c$) à l'aide d'un coefficient cultural (iii) d'une ET de la culture dans des conditions non standard ($ET_{pm}$) à l'aide du coefficient cultural et d'un facteur de sol (iv) d'une évapotranspiration basée sur la télédétection ($ET_{rs}$) à l'aide des une ou plusieurs données satellitaires d'observation de la Terre couvrant le champ et (v) d'une ET d'évapotranspiration de champ ($ET_{champ}$), dans lequel l'ET de la culture dans les conditions non standard est cultivée dans des conditions de gestion et environnementales qui diffèrent des conditions standard, dans lequel lorsque les cultures dans des champs s'écartent de l'$ET_c$ en raison de conditions non optimales incluant la présence de parasites et de maladies, la salinité du sol, la faible fertilité du sol, la pénurie d'eau ou l'engorgement du sol, alors le taux d'ET est réduit en dessous de l'$ET_c$ et l'étendue du stress biotique dû à une attaque par un parasite ou une maladie est calculée à l'aide d'images de caméra de smartphone de niveau de champ et d'un stress abiotique dû à la température, l'humidité est calculée à l'aide d'indices de végétation basés sur la télédétection, dans lequel l'ET basée sur la télédétection (*ETrs*) est estimée à l'aide d'une ou plusieurs données satellitaires comprenant des bandes multispectrales et infrarouges thermiques ;
à estimer un ruissellement à l'aide d'une méthode du numéro de courbe basée sur les une ou plusieurs données agrométéorologiques, et les une ou plusieurs données satellitaires d'observation de la Terre couvrant le champ ;
à calculer un bilan hydrique du sol du champ à l'aide de l'évapotranspiration de niveau de champ ($ET_{champ}$), du ruissellement, des informations de précipitations effectives, des données de capteur IoT, des une ou plusieurs caractéristiques de la culture et des une ou plusieurs propriétés de sol ;
à estimer les une ou plusieurs propriétés de sol et les une ou plusieurs caractéristiques de la culture à l'aide d'une approche de modélisation inverse, dans lequel les une ou plusieurs propriétés de sol incluent une capacité au champ, et les une ou plusieurs caractéristiques de la culture incluent le coefficient cultural ;
à estimer, selon une approche d'ensemble, l'humidité du sol au champ à l'aide du bilan hydrique du sol, et une humidité de sol estimée à l'aide des données de capteur IoT et une humidité de sol estimée à l'aide de données satellitaires radar à synthèse d'ouverture (SAR), dans lequel un estimateur d'ensemble de l'humidité du sol au champ ajuste dynamiquement un ou plusieurs paramètres utilisés pour une estimation du besoin en eau de la culture ; et
à estimer un besoin en eau de la culture du champ à l'aide (i) de l'humidité du sol au champ et (ii) d'une pluralité de paramètres de programmation d'irrigation comprenant un intervalle d'irrigation, une dose d'irrigation, un pourcentage d'épuisement admissible basé sur un coefficient d'épuisement.

**12.** Un ou plusieurs supports de stockage d'informations lisibles par machine non transitoires selon la revendication 11, dans lequel le besoin en eau de la culture du champ est estimé à l'aide des données d'irrigation, des informations de précipitations effectives, du ruissellement, d'une percolation, d'une remontée capillaire, d'une percolation profonde, et de l'évapotranspiration de référence ($ET_o$).

**13.** Un ou plusieurs supports de stockage d'informations lisibles par machine non transitoires selon la revendication 11, dans lequel la valeur d'humidité du sol au champ est estimée à l'aide d'un capteur d'humidité de sol basé sur un processus, de l'humidité de sol observée à l'aide des données satellitaires radar à synthèse d'ouverture (SAR), et de l'humidité de sol observée à l'aide des données de capteur IoT.

**14.** Un ou plusieurs supports de stockage d'informations lisibles par machine non transitoires selon la revendication 13,

dans lequel une technique de filtrage est appliquée sur la valeur d'humidité du sol au champ pour corriger des valeurs à venir d'humidité du sol au champ évitant une augmentation ou une diminution drastique des valeurs d'humidité de sol, et dans lequel l'humidité de sol est estimée pour un profil de sol horizontal et un profil de sol vertical.

**15.** Un ou plusieurs supports de stockage d'informations lisibles par machine non transitoires selon la revendication 11, dans lequel la modélisation inverse est appliquée pour une estimation de la capacité au champ et du coefficient cultural du champ à l'aide des valeurs d'humidité du sol au champ, dans lequel le stockage d'humidité de sol dans le profil de sol et le coefficient d'épuisement sont utilisés pour déclencher une rétroaction d'événement d'irrigation comprenant un temps d'irrigation dans le champ, et dans lequel le besoin en eau de la culture pour une journée consécutive est estimé sur la base de la rétroaction d'événement d'irrigation.

100
Satellite based earth observations
Weather forecast data
Internet and cloud based services
Smartphone
Grower/ Farmer
System 102
Irrigation estimation
Soil water balance
Crop growth modeling
Field level IoT enabled sensors
Region of interest
Example rice
Example cotton
Example tea
Example wheat
Example orange
Example corn
Weather station (agro-meteorological)

FIG. 1

System 102
Hardware Processor(s) 204
I/O Interface(s) 206
Memory 202
Modules 208
Evapotranspiration (ET) estimator unit 208a
Runoff estimator unit 208b
Soil water balance computation unit 208c
Ensemble based field soil moisture estimator unit 208d
Irrigation scheduling computation unit 208e

FIG. 2

Input
Crop characteristics
Field IoT sensor data
Agro-meteorological observation data
Weather forecast data
Satellite based earth observation data
Soil properties
Evapotranspiration estimator (208a)
Runoff estimator (208b)
Effective Rainfall
Reference crop evapotranspiration
Satellite based evapotranspiration
Irrigation system parameters
Ensemble crop evapotranspiration
Soil water balance computation unit (208c)
Process based soil moisture estimation
Ensemble based field soil moisture estimator (208d)
Soil moisture estimates using SAR satellite observations
A
B
C

FIG. 3A

A
B
C
Scheduling parameters
{Irrigation interval (i),
Depth of irrigation (d),
Percent allowable depletion
(delta)}
Irrigation depth (I)
Day of irrigation
No
Yes
Irrigation event
Switch on the irrigation
system for time (t)
Crop is under water stress
No need to irrigate for next
(x) days
Output
Event feedback
Irrigation scheduling computation unit (208e)

FIG. 3B

400

receive from a field a set of inputs comprising a IoT sensor data, one or more crop characteristics, one or more agro-meteorological weather data of the field, one or more satellite earth observation data covering the field, one or more soil properties of the field, a historical irrigation data, and a weather forecast data

402

estimate using the set of inputs (i) a reference crop evapotranspiration (ET) using the one or more agro-meteorological weather data and the IoT sensor data (ii) a crop ET under standard conditions ($ET_c$) using a crop coefficient (iii) a crop ET under non-standard conditions ($ET_{pm}$) using the crop coefficient and a soil factor (iv) a remote sensing based evapotranspiration ($ET_{rs}$) using the one or more satellite earth observation data covering the field and (v) a field evapotranspiration ET ($ET_{field}$)

404

estimate a runoff using a curve number method based on the one or more agro-meteorological weather, and the one or more satellite earth observation data covering the field

406

calculate a soil water balance of the field using the field level evapotranspiration ($ET_{field}$), the runoff, the effective rainfall information, the IoT sensor data, the one or more crop characteristics and the one or more soil properties

408

A

FIG. 4A

A
estimate the one or more soil properties and the one or more crop characteristics by using an inverse modelling approach, wherein the one or more soil properties includes a field capacity, and the one or more crop characteristics includes the crop coefficient
410
estimate an ensemble-based field soil moisture using the soil water balance, and a soil moisture estimated using the IoT sensor data and a soil moisture estimated using a synthetic aperture radar satellite data
412
estimate crop water requirement of the field using (i) the field soil moisture and (ii) a plurality of irrigation scheduling parameters comprising an irrigation interval, a depth of irrigation, a percent allowable depletion based on depletion coefficient
414

FIG. 4B

(a)
(b)
(c)
(d)
(e)
(f)
After irrigation or heavy rainfall soil profile reaches to field capacity and excess water flows as runoff
At field capacity t(FC)
Crop uses some water t(1)
Crop uses some water t(2)
Crop uses some water and some rain t(3)
At allowable depletion t(n)
At wilting point t(WP)
Air
Water
Solid
Regular increase or decrease in profile soil moisture. Crop not under moisture stress.
Crop under water stress
Crop under severe water stress
Trigger for irrigation event

FIG. 5

Evapotranspiration
Irrigation
Precipitation
Vertical
Zones
1
2
3
4
5
6
Runoff
Root Zone
Horizontal
Layers
Sub-soil
Percolation
Capillary rise
Deep Percolation

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 202421036177
- US 2022061236 A1
- US 2021110157 A1